# EUROPEAN PATENT APPLICATION

(11) **EP 2 161 043 A1**
(43) Date of publication of application: **10.03.2010**
(21) Application number: 08163816.5
(22) Date of filing: 05.09.2008
(51) Int. Cl.: A61L 27/34, A61L 27/50, A61F 2/00, A61F 2/12

(54) **Coated medical device and method of coating a medical device**

(71) Applicant: ECOLE POLYTECHNIQUE FEDERALE DE LAUSANNE (EPFL), 1015 Lausanne (CH)
(72) Inventor: Majd, Hicham, 1020, Renens (CH); Pietramaggiori, Giorgio, 1205, Genève (CH); Hinz, Boris, 1053, Bretigny-sur-Morrens (CH)
(74) Representative: Grosfillier, Philippe

(57) **Abstract**

The medical device comprises a surface coated with cell-adhesive proteins in the form of a micro pattern.

## Description

### FIELD OF THE INVENTION

The present invention relates to a coated medical device and a method of coating the same.

### BACKGROUND OF THE INVENTION

Medical devices in contact with living cells and tissues induce a foreign body reaction due to their physical and chemical properties. This process results in a capsule formation around the implant that often contracts and results in malfunctioning of the device and clinically relevant complications such as pain and dysmorphysm of the patient that ultimately require additional surgical operations.

The reaction of cells to a foreign material such as for example a silicone breast implant, hearth valve, joint and bone prosthesis, etc., which cannot be phagocytated, enzymatically digested or otherwise eliminated, is the formation of fibrotic capsule around it. This problem is exemplarily described by Anderson JM, Rodriguez A, Chang DT: Foreign body reaction to biomaterials, Semin Immunol 2008, 20:86-100.

This capsule is the result of a chronic inflammatory process around the material that resolves only after the formation of the capsule itself (see e.g. Shanklin DR, Smalley DL: Dynamics of wound healing after silicone device implantation, Exp Mol Pathol 1999, 67:26-39). Similarly to any healed wound, the capsule is created by the myofibroblasts that deposit collagen fibers around the foreign material and eventually contract it (see Li AG, Quinn MJ, Siddiqui Y, Wood MD, Federiuk IF, Duman HM, Ward WK: Elevation of transforming growth factor beta (TGFbeta) and its downstream mediators in subcutaneous foreign body capsule tissue, J Biomed Mater Res A 2007, 82:498-508; Suska F, Emanuelsson L, Johansson A, Tengvall P, Thomsen P: Fibrous capsule formation around titanium and copper, J Biomed Mater Res A 2008, 85:888-896; and Coleman DJ, Sharpe DT, Naylor IL, Chander CL, Cross SE: The role of the contractile fibroblast in the capsules around tissue expanders and implants, Br J Plast Surg 1993, 46:547-556).

It has been reported by Prantl L, Schreml S, Fichtner-Feigl S, Poppl N, Eisenmann-Klein M, Schwarze H, Fuchtmeier B (Clinical and morphological conditions in capsular contracture formed around silicone breast implants, Plast Reconstr Surg 2007, 120:275-284) that in up to 20-30% of implanted prosthesis, capsule formation is excessive and leads to clinically relevant, painful contractures around the device. This process significantly shortens the lifespan of the device, its function and dramatically reduces the quality of life of the patients. Patients with excessive capsular formation need to receive a second operation to remove the capsule around the device, which considerable increases in the morbidity and costs (see Minami E, Koh IH, Ferreira JC, Waitzberg AF, Chifferi V, Rosewick TF, Pereira MD, Saldiva PH, de Figueiredo LF: The composition and behavior of capsules around smooth and textured breast implants in pigs, Plast Reconstr Surg 2006, 118:874-884). In addition, the likelihood of capsule formation increases with the operations (see also Prantl L, Schreml S, Fichtner-Feigl S, Poppl N, Eisenmann-Klein M, Schwarze H, Fuchtmeier B: Clinical and morphological conditions in capsular contracture formed around silicone breast implants, Plast Reconstr Surg 2007, 120:275-284).

The problem of capsule formation has also been discussed in patent literature. In US 4,772,285 a collagen coated soft tissue prosthesis is described for reducing capsule formation. While this patent proposes a strategy to decrease capsule formation, no mechanism is provided to effect myofibroblast formation. Further the clinical evidence is lacking.

US 4,955,907; 4,731,081; 5,571,183; 5,207,709; 5,354,338; 4,428,082 and 4,298,998 also propose solutions to avoid or diminish capsule formation.

All these documents have several common denominators, which have the potential of making them unsuitable for resolving this problem in human beings.

For example US 4,298,998 discloses causing a capsule to form at a predetermined, controlled distance from the surface of the implant, thus resulting in the same capsule but at a different location. The end result clinically appears to be a hard capsule for the patient and not resolving the problem.

Similarly, the implant described in US 5,207,709 includes a plurality of fine projections extending from the outer surface arrayed in a basket weave-like, herringbone-like, or other suitable pattern to create a sinuous path for collagen formation around the implanted device. It appears that this implant actually creates or invites collagen formation again in another location around the implant and again therefore is not resolving the problem.

Still other patents relate to the implant being surrounded by a medical grade elastomer or as described in US 4,944,749 a viscous gel coating with the membranes constructed of a suitable material such as medical grade silicone rubber which does not react with human tissue. The outer membrane contains an amount of viscous gel, for example a silicone rubber gel of medical grade silicone.

It appears in the end that this patent still has a silicone tissue interface that has accounted for problems.

US 4,610,690 is directed to an implant with a lubricious layer of an acrylamide polymer radiation bonded to at least one wall surface of a silicone shell or bag. Potential long-term effects in human beings of an acrylamide polymer interface are not discussed.

All these aforementioned patents continue to have unnatural chemicals as interface with human tissue, which is exactly what patients do not want in their body and what usually causes problems.

In US 4,995,882 an organic fill solution is proposed to solve the problem. The implant is proposed the use a triglyceride fill substance such as peanut oil or sunflower seed oil as a filling substance.

Although some implants of this kind were implanted in Europe, they were never authorized for implantation in the United States and were subsequently taken off the market worldwide because of various problems.

Although many strategies have been developed to reduce capsule formation, from modifications of the surface of the implants to chemical coatings, only mixed results have been reported and, with the increase in the demand for implantable devices, the clinical problem is on the rise.

Myofibroblasts play a main role in capsule formation and contraction in healing tissues as it is described by Hinz B, Phan SH, Thannickal VJ, Galli A, Bochaton-Piallat ML, Gabbiani G (The myofibroblast: one function, multiple origins, Am J Pathol 2007, 170:1807-1816).

Further the myofibroblast behavior during healing was described and then extensively studied by Hinz B (Formation and function of the myofibroblast during tissue repair, J Invest Dermatol 2007, 127:526-537) and strategies were developed to effectively modulate the formation and function of myofibroblats. One of these strategies has been to use silicone substrates 80 micrometers thick produced by mixing PDMS curing agent and base (Sylgard 184, Dow Corning), in ratios between 1:5 and 1:80. Young's modulus was determined according to Balaban et al and confirmed with AFM.

Micropatterned silicone substrates were created similar to a published method, with important modifications. The substrates were produced with an elastic modulus of approximately 23 kPa to promote the formation of supermature focal adhesions (su-FAs) and alpha-SMA stress fiber incorporation, which was not achieved using 12 kPa substrates. On these silicone substrates, PDMS stamps for microcontact printing were obtained from silicon wafer molds and incubated for 30 minutes with different protein solutions (10-100 microg/ml), quick-dried, and put in conformal contact with substrates for 1 minute. Non-printed regions were passivated with 0.1 mg/ml PLL-g-PEG. For PLL-g-PEG stamping, both the substrate and the stamp were treated for 15 seconds with oxygen plasma. To provide stretchable PDMS membranes with adhesion islets, a method was used of microcontact printing. (See Balaban NQ, Schwarz US, Riveline D, Goichberg P, Tzur G, Sabanay I, Mahalu D, Safran S, Bershadsky A, Addadi L, Geiger B: Force and focal adhesion assembly: a close relationship studied using elastic micropatterned substrates, Nat Cell Biol 2001, 3:466-472; Goffin JM, Pittet P, Csucs G, Lussi JW, Meister JJ, Hinz B: Focal adhesion size controls tension-dependent recruitment of alpha-smooth muscle actin to stress fibers, J Cell Biol 2006, 172:259-268; Singhvi R, Kumar A, Lopez GP, Stephanopoulos GN, Wang DI, Whitesides GM, Ingber DE: Engineering cell shape and function, Science 1994, 264:696-698; and Csucs G, Michel R, Lussi JW, Textor M, Danuser G: Microcontact printing of novel co-polymers in combination with proteins for cell-biological applications, Biomaterials 2003, 24: 1713-1720).

### SUMMARY OF THE INVENTION

It is therefore an object of the invention to provide a medical device and a method to produce it with which the capsule formation in the body can be further diminished or even avoided.

This can be achieved by a medical device wherein a surface of the medical device is coated with cell-adhesive proteins in form of a micro pattern.

According to the invention it is provided an appliance comprising a coating based on a micro patterned area of a medical device surface.

This coated surface which is supposed to come in contact with living cells and tissues to modulate cell adhesion is composed from two specific regions:
1. Micro-area (islands) where cells (tissue) can attach, and
2. Other regions where cells (tissue) cannot attach.

This invention relates in general to a method and device for guiding cellular adhesion on medical devices. The invention relates to a method comprising micro patterned surfaces to coat devices (implantable and external) in contact with any cell and tissue of the body.

Specifically, the invention contemplates the use of such technique in combination with devices implanted or externally applied to the body. This proposal outlines a novel strategy based on micro patterned surfaces to decrease the formation of a capsule and contraction around medical devices.

According to a preferred embodiment of the invention the medical device comprises silicone.

Other preferred embodiments include the use of plastic, metal, Teflon, ceramics, epoxit and/or collagen as a material of the medical device.

According to one embodiment of the invention the medical device according to the invention comprises micro pattern where the arrays show uniform geometric shapes.

Another possibility would be the use of different geometric shapes.

The distance between the arrays is preferably from 0.01 to 100 µm and the area of the arrays is from 0.01 to 100 µm².

In another embodiment of the invention good results could be achieved when the distance between the arrays was about 5 µm and the area in the range of 4 to 8 µm².

In a preferred embodiment of the invention the medical device further comprises a substrate.

The substrate which is preferably applied to the medical device before coating same with proteins could be silicone, plastic, bio resistant materials, etc..

Further the invention describes a method of coating a medical device, wherein the medical device is coated with cell-adhesive proteins in form of a micro pattern.

As already mentioned it could be in some embodiments of advantage if the medical device is coated by a substrate before being coated by the proteins.

According to one embodiment of the invention the proteins are applied to the medical device by a stencil.

For applying the proteins according to another embodiment the stencil could be brought in contact with the surface of the medical device and the proteins are fixed to the surface through holes in the stencil.

The fixing of the proteins to the medical device could be done by every possibility known from the art. As examples, but not restricted to it, it should be mentioned covalent binding, electro deposition or precipitation of proteins on the medical device and/or the substrate.

The medical device as described can be used for example, but not restricted to it, for breast implants, tissue expanders, inflatable pumps, implants, transplants, prosthesis, insulin pumps, drug delivery systems, cardiovascular devices, skin substitutes, wound dressings and/or tubes.

### DETAILED DESCRIPTION OF THE INVENTION

These and other objects, features, aspects and advantages of the present invention will become more apparent from the following detailed description of the present invention when taken in conjunction with the accompanying drawings.
Figure 1 shows a medical device before and after being coated according to one embodiment of the invention; and
Figure 2 shows examples for the array of the micro pattern according to a preferred embodiment of the invention.

The invention describes a new method of micro-deposition of cell-adhesive proteins and molecules onto the surface of medical devices, such as, but not limited to highly deformable and elastic substrates (such as, but not limited to silicone), Titanium, Plastic, Polyurethane and generally all materials used for medical devices and implants.

The micro-deposited array of proteins guides cell adhesion on medical devices with the objective to reduce the fibrotic reaction of the cells and tissues in contact with the device are described further in detail.

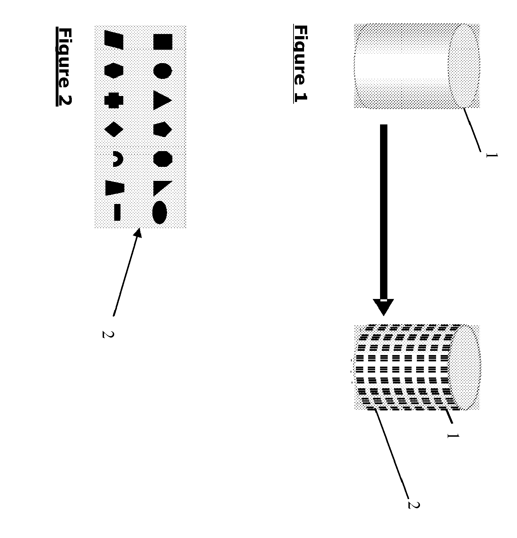

As shown in Figure 1 according to an embodiment of the invention a microperforated stencil or mask with arrays of specific area and geometric distribution is used to micro-transfer proteins to coat a medical device 1. Deposition of the proteins and molecules is in the form of micro-arrays 2, with any possible geometrical shape and spatial organization and individual area. Arrays 2 are deposited to modulate cell and tissue adhesion to medical devices 1.

The distance between stamped arrays according to a preferred embodiment of the invention will be from 0.01 µm to 100 µm. The area of the printed surfaces preferably will range from 0.01 µm to 100 µm².

The printed arrays 2 could have any geometrical shape. Examples to which the invention is not limited are illustrated in Figure 2.

Results according to one embodiment of the invention showed good anti-fibrotic effects using an area of 4-8 µm² and a distance between arrays of about 5 µm.

A mask or a stencil is micro fabricated using technology as for example, but not restricted to it, photolithography, dry and wet etching, laser cutting.

As type of mask or stencil exemplatory, but not restricted to it, should be mentioned soft stencil made from silicone and flexible polymers and hard stencil from silicon, hard polymer and metal.

To deposit proteins onto medical device as for example an implant surface, the stencil is first brought in conformal contact with the surface and then the proteins are fixed on the surface through the micro-holes of the stencil by covalent binding using 3-aminopropyltriethoxysilane (APTES) and glutaraldehyde or formaldehyde. After any possible chemical crosslink of proteins, the stencil is removed and the pattern remains on the implant surface.

Another possible method is electro deposition or precipitation of proteins on implant or medical device surface through the micro-holes of stencil. These possibilities are just examples and should not limit the invention to this approach of deposition.

Silicone pads (1x1 cm) coated on both side with an optimized micropatterned deposition of the protein fibronectin, as in the methods described above, were inserted in subcutaneous pockets, in female Wistar rats (250-350 g). On the scapular region of the dorsum of these animals, 4 coated silicone pads were placed. Each animal received four implants: one coated implant with optimized micropatterned deposition of protein; one control implant with full collagen coating as in U.S. Pat. No. 4,772,285; and two non-coated silicone implants, alternating the location on successive animals.

The micropatterned surface can be applied to any medical device such as, but not limited to:
- Silicone breast implants
- Tissue expanders and inflatable pumps
- Bone, and cartilage and orthopaedic implants
- Tendon, nerve, and ligament transplants, substitutes and implants
- Implantable pumps, such as insuline pumps and drug delivery systems
- Cardiovascular devices, such as pace makers, vascular prosthesis, hearth valves, vascular stents
- Skin substitutes and cell scaffolds
- Wound dressings, including dressings and wound interfaces connected to a vacuum (negative pressure dressings)
- Acoustic implants
- Ear, throat, nose and eye implants
- Brain, central and pheripheral nervous system implants and prosthesis
- Tubes, connecting tubes, drainage tube systems

## Claims

1. Medical device comprising a surface coated with cell-adhesive proteins in the form of a micro pattern.

2. Medical device according to claim 1, wherein the surface comprises a substrate.

3. Medical device according to any of the foregoing claims, wherein the device comprises silicone and/or plastic and/or metal and/or collagen.

4. Medical device according to any of the foregoing claims, wherein the micro pattern comprises arrays with uniform geometric shapes.

5. Medical device according to any of the foregoing claims, wherein the micro pattern comprises arrays with different geometric shapes.

6. Medical device according to any of the foregoing claims, wherein a distance between the arrays is from 0.01 to 100 µm and an area of the arrays is from 0.01 to 100 µm².

7. Medical device according to any of the foregoing claims, wherein the distance between the arrays is about 5 µm and the area is from 4 to 8 µm².

8. Method of coating a medical device, wherein the medical device is coated with cell-adhesive proteins in form of a micro pattern.

9. Method according to claim 8, wherein the medical device is coated by a substrate before being coated with the proteins.

10. Method according to any of the claims 8 and 9, wherein the proteins are applied by a stencil.

11. Method according to any of the claims 8 to 10, wherein the stencil is brought in contact with the surface of the medical device and the proteins are fixed to the surface through holes in the stencil.

12. Method according to claim 11, wherein the fixing is done by covalent binding.

13. Method according to claim 11, wherein the fixing is done by electro deposition.

14. Use of the medical device according to any of the claims 1 to 7 for breast implants, tissue expanders, inflatable pumps, implants, transplants, prosthesis, insulin pumps, drug delivery systems, cardiovascular devices, skin substitutes, wound dressings and/or tu bes.
